Europäisches Patentamt

(19) European Patent Office

Office européen des brevets

(11) EP 0 743 323 A1

(12) **EUROPEAN PATENT APPLICATION**

(43) Date of publication:
20.11.1996 Bulletin 1996/47

(51) Int. Cl.$^6$: **C08B 30/18**, A61K 31/715, A61K 7/48

(21) Application number: 95107578.7

(22) Date of filing: 18.05.1995

(84) Designated Contracting States:
AT BE CH DE DK ES FR GB GR IE IT LI LU MC NL PT SE

(71) Applicant: FARMACEUTICI DAMOR S.p.A.
I-80145 Napoli (IT)

(72) Inventor: Riccio, Rodolfo
I-80145 Napoli (IT)

(74) Representative: Minoja, Fabrizio
Studio Consulenza Brevettuale,
Via Rossini, 8
20122 Milano (IT)

(54) **Saccharide fraction obtainable from starch, its use as active principle in the treatment of wounds and ulcers and pharmaceutical compositions containing it**

(57) A description is given of a fraction obtainable from starch, possessing a molecular weight between 3000 and 30 000 daltons, which is active in the treatment of wounds and ulcers.

EP 0 743 323 A1

## Description

The present invention relates to a fraction obtained from starch that has therapeutic activity and can be used in the treatment of wounds and ulcers.

Growth factors are currently indicated for their efficacy in the treatment of wounds and ulcers, as well as in all pathological situations requiring control and/or acceleration of cell proliferation.

With regard to growth factors, it must be emphasized that the high industrial costs of the extractive process (owing to the extremely low yields obtained with the processes of purification from the animal starting material), the risk of contamination by viruses and other animal proteins, considerable instability, variability of chemical structure depending on the organ and on the animal species from which the process starts, and above all the high risks connected with their proteinaceous nature (anaphylaxis, allergies, hypersensitivity etc.), drastically limit their therapeutic use.

On the other hand the clinical use of macromolecules of glycidic nature (dextrans, hyaluronic acid, other GAGs) as well as extracts of numerous plant species (such as Aloe, Centella asiatica etc.), has been well established for some time, and they are widely used for all pathologies affecting the epidermal tissue.

The object of the present invention is to provide an active principle with saccharide structure for the treatment of all kinds of ulcers and wounds, and which can also be used in all pathological situations in which control and/or acceleration of cell proliferation is required. This active principle must be free from the disadvantages inherent in substances of animal origin, must be easily available and of low cost by means of a simple and repetitive process.

## SUMMARY OF THE INVENTION

It was found that a particular fraction obtained from starch shows, surprisingly, some biological characteristics that are typical of growth factors, though being clearly different from them in that it is of vegetal instead of animal origin, and that it has a saccharide instead of protein chemical structure.

Thus, the present invention comprises a fraction obtained from starch possessing molecular weight between 3000 and 30 000, its use as active principle for the preparation of a medicament that can be used for the treatment of wounds and ulcers and pharmaceutical compositions which contain the said active principle.

Detailed description of the invention

The fraction according to the present invention can be obtained starting from a starch of any origin (potato, rice, wheat, durum and common wheat, maize or Indian corn, other Gramineae, etc.) and with any degree of purity (and hence also from flour, bran, whole or crushed or germinated seeds etc.).

According to the present invention, the process envisages the preparation of a raw intermediate extract obtained by mild acid hydrolysis of starch and the subsequent purification of the active fraction.

The said process comprises the following stages:

a) hydrolysis of the starch in an aqueous solution at a pH of between 2 and 7 at a temperature between 100 and 125°C;

b) elimination of the solid residue until a clear first solution is obtained;

c) elimination of the fraction with molecular weight greater than 30 000 daltons from the said solution, to give a second solution;

d) isolation of the fraction with molecular weight between 3000 and 30 000 from the said second solution.

According to a first embodiment of the invention, the starch can if necessary be moistened with heat-sterilized distilled water prior to stage a).

Stage a) is preferably carried out at a pH between 2 and 4. Hydrolysis is effected by conventional techniques, preferably in an autoclave, if necessary after maceration for 2 or 3 days.

In a second embodiment of the invention, elimination of the solid residue in stage b) can be effected by an enrichment process. In this case, the filtrate obtained in stage b) has whole starch added to it and the hydrolysis of stage a) is repeated. Higher yields are obtained in this way.

The solution obtained from stage b) can be sterilized and, when suitably diluted, can be used for the next stage.

Elimination of the fraction with molecular weight greater than 30 000 (stage c)) can be effected by a conventional method known to a person skilled in the art, for example gel filtration or ultrafiltration.

Similarly, the fraction of interest is isolated by means of the conventional techniques mentioned above.

In its final form, the fraction that is the subject of the present invention can be dried or lyophilized by conventional methods.

The starch fraction according to the present invention (hereinafter also denoted by the symbol SF) will now be described in greater detail with reference also to the following figures, in which:

Fig. 1 is a chromatographic diagram of the fraction of interest after it was isolated;

Fig. 2 is a chromatographic diagram of the solution obtained after hydrolysis and filtration (stage a) and b)).

The behaviour in ultrafiltration on the laboratory scale confirms what was found on an industrial scale, namely that the fraction SF is retained by the membrane with cut-off at 3000, and eluted by the membrane with cut-off at 30 000.

It should be borne in mind, however, that the cut-offs of ultrafiltration membranes have a purely theoretical value, being referred to globular proteins; the behaviour of a saccharide component can therefore reasonably be different, and accordingly the result obtained is only to be regarded as a guide.

For chromatographic characterization of the fraction SF, HPAE (high-performance anion exchange chromatography) was employed using a liquid chromatograph equipped with a pulsed electrochemical or amperometric detector (PAD/PED). This type of chromatographic system is specific not only for organic and inorganic anions already free in solution, but also for carbohydrates because at extremely alkaline pH, sugars behave as negatively charged species, with a net charge that is strictly dependent on chemical structure, molecular weight, and of course the presence of polar groups; therefore, by using special columns with pellicular resins with eluents with high pH, and in particular a detector that is extremely sensitive and selective for anions, it is possible to detect sugars even at very low concentrations, in contrast to other detectors that are less sensitive (UV, refractometers etc.), hence using a chromatographic scheme of the same simplicity as normal ion-exchange chromatography.

Procedure

- Chromatographic column: Carbopack PA1 (4 x 250 mm) with Carbopack PA1 Guard pre-column (10-32) from Dionex.
- Eluent system:
  Eluent pump A: 1M Sodium acetate in 0.5 M NaOH
  Eluent pump B: 0.5 M NaOH

The gradient is set by the following programme (where the flow rate is expressed in ml/min):

| Gradient programme | | | | |
|---|---|---|---|---|
| Step | Minutes | Flow rate | %A | Curve |
| 0 | 12 | 1 | 5 | -- |
| 1 | 10 | 1 | 15 | +1 |
| 2 | 15 | 1 | 15 | -- |
| 3 | 10 | 1 | 20 | 0 |
| 4 | 10 | 1 | 30 | 0 |
| 5 | 10 | 1 | 35 | 0 |
| 6 | 15 | 1 | 100 | +1 |
| 7 | 5 | 1 | 100 | -- |

- Detector: PAD from Dionex equipped with gold electrode, in the following operating conditions:

| Detection programme | |
| --- | --- |
| Time (sec) | E (volt) |
| 0.00 | +0.10 |
| 0.50 | +0.10 |
| 0.51 | +0.60 |
| 0.59 | +0.60 |
| 0.60 | -0.60 |
| 0.65 | -0.60 |

Integration time: 0.3-0.5 seconds

| Analog 1: | 1 volt | Mode: | Integrated amperometry |
| --- | --- | --- | --- |
| SW 1: | standard | SW 3: | 5 |
| SW 2: | standard | SW 4: | 3 On (only ref.) |
| Scale: | 300 nanocoulomb | | |

- Integrator: Perkin-Elmer LCI 100, with the following operating conditions:

| Jumper range select | 10 V | Peak width | - 7 |
| --- | --- | --- | --- |
| Attenuation | 64 | B code | 0 |
| End time | 55 | Offset | 5 |
| Chart | 5 | | |

- Injection valve: Rheodyne Mod. 7125 equipped with Rotor Seal in Tefzel and 100 µl injection loop.
- Injection volume: 75 µl of a solution of SF at concentration of about 20 mg/100 ml of water.

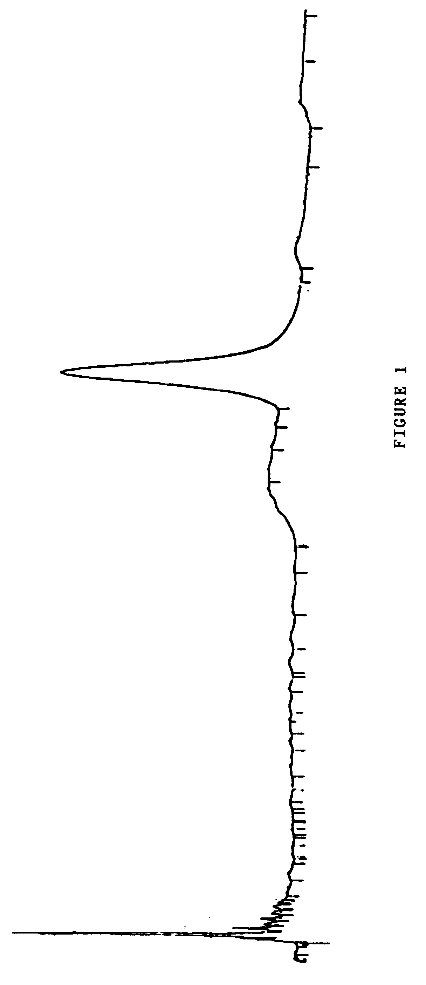

The chromatogram obtained (see Fig. 1) shows a single broad peak with an Rt of about 44 minutes.

This represents an unequivocal way of identifying the fraction SF.

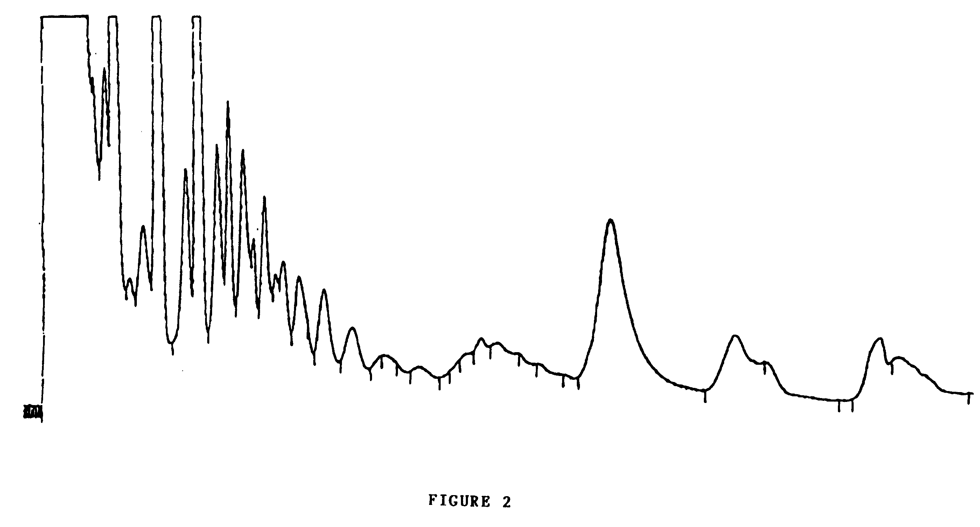

Fig. 2 shows the chromatogram of the initial extract (obtained in the same chromatographic conditions, by injecting 75 µl of a solution that has a total dry yield of 200 mg/ml), which is also characteristic and is intended as a "finger-print"; the peak relating to SF can of course be seen among the others.

For quantitative determination of the glycidic components (in toto), a colorimetric method was adopted, using the reagent orthotoluidine, which specifically transforms the reducing hexoses into a blue-coloured complex, which can be determined easily.

Analysis is effected on the sample after complete acid hydrolysis, using pure glucose as the standard.

It should be emphasized that this analysis does not permit qualitative differentiation of the hexoses making up the saccharide chain, being nonspecific, in the sense that the coloured complex is formed irrespective of the nature of the reducing hexose (glucose, galactose, mannose etc.) albeit with a different specific extinction coefficient.

Procedure

A solution of SF 100 mg/litre in 0.35% HCl is prepared, and hydrolysed for 1 hour at 1 atmosphere (121°C).

A solution of pure glucose 100 mg/litre in water is prepared. To 1 ml of each solution, placed in a test-tube with a screw stopper, are added 9 ml of orthotoluidine reagent (consisting of 3% orthotoluidine in acetic acid, containing 0.075% of thiourea); the mixture is left on a boiling water bath for 8 minutes, then cooled, and the intensity of the blue coloration is measured against water with a spectrophotometer at 630 nm.

At least 70% of the weight of the fraction SF is made up of reducing hexoses.

It should be remembered that the treatment of complete hydrolysis, effected on the first analysis sample, can be partially destructive; therefore the result (70%) should be regarded only as a minimum value.

Qualitative analysis of the constituent sugars is effected by HPAE (high-performance anion exchange) chromatography, after hydrolysis of the fraction SF.

For details on the use of HPAE for analysis of sugars and the apparatus used, see the description for the chromatographic behaviour relating to the whole (i.e. non-hydrolysed) fraction SF.

Procedure

Hydrolysis is effected with 0.35% (v/v) HCl at 100°C for 20 hours, on a sample of SF in solution at a concentration of about 75 mg/100 ml.

The conditions of analysis in HPAE are:

- Carbopack PA1 column (4 x 250 mm)
- Isocratic eluent 0.017 N NaOH
- Constant flow rate of 1 ml/min
- PED detection
- Injection volume: 75 µl of the solution of hydrolysed sample, diluted 1 to 100 with water.

Similar analysis is effected on the commonest monosaccharides, as a standard.

The sample under examination is found to have massive and predominant presence of glucose, whereas other peaks, relating to other hexoses (mannose, galactose), hexosamines or pentoses are present only in much smaller amounts.

A similar test effected on the unhydrolysed sample showed absence of free glucides (their presence is in any case excluded by the method of obtaining the SF by ultrafiltration, which eliminates molecules with molecular weight less than 3000 daltons), demonstrating that the carbohydrates present in SF form a complex structure of the polymeric type.

The fraction according to the present invention is endowed with activity similar to that of the growth factors of animal origin, and therefore it can be used as active principle in the manufacture of a medicament for the treatment of wounds and ulcers.

The therapeutic activity is illustrated below by various pharmacological trials.

## A. Test of healing in vivo on experimental wounds

The healing test in vivo, effected on animals either with application by injection or with topical application, demonstrates the true usefulness of the product in the treatment of wounds.

Procedure

The test is effected on male rats of the Wistar strain weighing between 220 and 250 g. For a week before the test the animals are kept in controlled conditions of temperature, humidity and light, with free access to food and water.

The animals are subdivided into homogeneous experimental groups each comprising 10 animals; one group is used as a control and is treated with a placebo, the other group is treated with the product.

On the morning of the first day of testing, all the animals undergo a surgical procedure to produce a standard wound, obtained in the following way: after mild anaesthesia (10% ethylurethane at a dose of 10 ml/kg), the dorsolumbar region of each animal is carefully shaved; after disinfection, the skin is cut around the edge of a metal disk with diameter of 2.5 cm (0.5 in the case of topical treatment), and then the skin and the subcutaneous tissue are removed using curved scissors. Substantially identical wounds are obtained for all the animals.

After each day of treatment, the wound is suitably dressed and the animals are kept in individual cages.

The extent of the wounds is measured with a planimeter (having traced the wound on a sheet of transparent paper) every day for 9 days (5 days in the case of topical treatment).

Treatment (daily) by injection

The animals of the control group are treated with an intraperitoneal injection of placebo, consisting of physiological saline (0.9% NaCl) at dose of 1 ml for every 100 g of body weight.

The animals of the other group are treated with an intraperitoneal injection of the product dissolved in physiological saline (NaCl 0.9%) at a concentration of 1 mg/100 ml, at dose of 1 ml for every 100 g of body weight.

Treatment (daily) by topical application

The wounds of the animals of the control group are treated with sterile gauze impregnated with placebo, consisting of physiological saline (NaCl 0.9%).

The wounds of the animals of the other group are treated with sterile gauze impregnated with the product, dissolved in water at a concentration of 20 mg/100 ml.

Results

In the animals treated with the product, an acceleration of the repair process is observed, manifested by a marked reduction of the areas of the wounds relative to the control group, on average by about 20-30% at the end of the treatment.

## B. Test for stimulation of cellular growth of fibroblasts

Fibroblasts are cells that perform a fundamental role in the mechanism of tissue repair. The test is therefore carried out to verify whether the healing effect of the product occurs as a result of a mechanism of stimulation of cellular growth of these organisms.

Procedure

Cultures of fibroblasts of mice of the 3T3 line are maintained in a DME medium supplemented with 10% v/v of calf serum (CS), penicillin (10 U/ml). These cultures are grown in 25-ml Falcon bottles at 37°C in a humid atmosphere containing 5% $CO_2$ and subcultured every 4-6 days. The confluent cultures are then washed with 3 ml of PBS and are trypsinized with 0.25% trypsin at 37°C. To deactivate the trypsin, 2 ml of DME is added and the cellular pellet obtained after centrifugation is diluted with fresh culture medium until a suitable seeding density is reached.

The stimulating effect of the product is tested on fibroblasts that have been made quiescent by passaging through a medium containing low concentrations of CS. The cells are seeded at a density of about 2000-4000 cells per pocket in DME supplemented with 5% of CS. The medium is renewed after 18 hours and replaced with fresh medium containing 0.6% CS, and the cells are left to grow for a further 48 hours before each subsequent test of activity.

For the test in question, the product (diluted in water at a concentration of 20 mg/100 ml) is added, at increasing concentrations between 2 and 20% v/v, every day throughout the period of growth.

At the end of the fifth day, the number of cells is estimated by trypsinizing the cultures and counting the cells in a Coulter counter.

Results

Addition of the product at the stated dilution produces a marked stimulation of cell growth, in a dose-dependent manner. The effect begins to be significant at a percentage of 5% of product (diluted as indicated above), and is maximum between 10 and 20%, in a slightly smaller quantity than that exhibited by the CS at equal concentration, but at least 4 times greater than that of a control, at the maximum point.

## C. Test of activation of ODC (ornithine decarboxylase)

Ornithine decarboxylase is the key enzyme in the synthesis of the polyamines spermine and spermidine, catalysing the conversion of the amino acid ornithine to putrescine. These polyamines are involved in control of the processes of cell multiplication.

The test is carried out to verify whether the stimulating action of the product on growth of fibroblasts correlates with the increase of activity of ODC.

Procedure

The activity of ODC is tested in 3T3 cells grown in a medium containing 0.6% CS (calf serum), and prepared as described above in the test of stimulation of the growth of fibroblasts.

The activity of ODC is measured by the method described by Russell [Proc. Natl. Acad. Sci. USA 60 1420 (1968)], 6 hours after adding the product (diluted in water to a concentration of 20 mg/100 ml) at quantities increasing from 1% to 10% v/v.

Results

The product, at the concentrations and in the conditions tested, shows, 6 hours after addition, a marked stimulation of ODC, of about 4 times greater than that of a control, and similar to that of CS at equal concentration, in a dose-dependent manner.

**D. Test of stimulation of the hydrolysis of inositol phospholipids**

It is now amply documented that one of the principal biochemical mechanisms by which a substance is able to activate the processes responsible for cell activation and multiplication consists of the specific stimulation of the hydrolysis of membrane inositol phospholipids, which in its turn produces an increase in two second messengers, inositol trisphosphate (InsP3) and diacylglycerol. This mechanism is intimately connected with, among other things, another system of signal transduction, activation of the tyrosine kinase responsible for example for the action of EGF (epidermal growth factor), of insulin and of insulin-like factors.

It was therefore considered to be useful to evaluate the effect of the product on the accumulation of inositol phosphates (as a specific index of the hydrolysis of phosphoinositides) in 3T3 cells, both in basic conditions and after stimulation with CS serum, which is a known activator of the metabolism of phospholipids in fibroblasts.

Procedure

The hydrolysis of inositol phospholipids is tested in 3T3 cells grown in a medium containing 0.6% CS (calf serum), and prepared as described above in the test of stimulation of the growth of fibroblasts.

Once quiescence is attained, the cells are washed with Ham's F-10 medium (with low concentration of inositol), and incubated at 37°C for 24 hours with addition of CS at 0.6% and 0.6 micromole of 2-[$^3$H]myoinositol for 24 hours, to label the membrane inositol phospholipids. At the end of this incubation, the cells are washed with Krebs-Henseleit buffer containing 10 mmol LiCl and 0.1% BSA.

The cells are then incubated for 24 hours with amounts increasing from 0 to 20% (v/v) of product (in aqueous solution at a concentration of 4 mg/100 ml), with and without CS at 10%.

The inositol phosphates are then assayed by the following technique: the incubation medium is aspirated off, and the cells are extracted with chloroform/methanol/water 1/1/1 (v/v); after centrifugation, the aqueous phase is taken and loaded onto 1 ml columns of Dowex-1 in formate form; the columns are washed with 24 ml of water to remove the labelled inositol that has not been incorporated; the inositol phosphates are then eluted by washing with 0.2 M ammonium formate and 0.1 M formic acid.

The radioactivity is then determined by scintillation spectrometry.

Results

The product, at the concentrations and in the conditions tested, displays marked stimulation of hydrolysis of inositol phospholipids, in a dose-dependent manner, of about 4 times greater than that of a control, and similar to that of CS at equal concentration.

It should be noted that simultaneous presence of the product and of CS leads to greater activation than with CS alone (and with the product alone), suggesting that there is an interesting synergic effect between the two components.

**E. Test of activity on induced gastric ulcers**

To assess the effects of the product on gastric ulcers, tests were conducted in vivo on laboratory animals, in which ulcers are induced by the use of stomach-damaging substances such as reserpine, serotonin and phenylbutazone: in a fasting rat these cause stomach-damaging effects, mainly haemorrhaging, which develop rather gradually, reaching a maximum 4-6 hours after treatment.

From the viewpoint of pathological anatomy, the lesions are characterized by diffuse hyperaemia of the gastric mucosa (through intense vasodilatation and congestion of the microcirculation) associated with haemorrhagic areas and presence of necrotic-haemorrhagic and punctiform ulcers in the body of the stomach, the antrum and the duodenum.

In particular, from the pathogenetic standpoint, the ulcerative-haemorrhagic lesions induced by reserpine are correlated with:

- release of histamine by the mast cells and by the HFC (histamine forming capacity) cells of the stomach wall
- release of serotonin from the enterochromaffin cells.

Procedure

The investigation was conducted on 6 separate groups of 6 male Sprague-Dawley rats weighing 150-170 g, kept in individual metabolism cages and fasted for the 36 hours preceding the tests, with free access to water.

In the two groups relating to ulcers induced by phenylbutazone, this was injected intraperitoneally at a dose of 140 mg/kg (in 10 ml/kg of a 0.5% solution of carboxymethylcellulose + 3 drops of Tween 80).

1. In the case of the groups with ulcers induced by reserpine or by serotonin, the product was administered (in both groups) at doses of from 1 to 4 mg/kg, in physiological saline (10 ml/kg); in parallel, the control groups received physiological saline (10 ml/kg).

Administration was effected by the intraperitoneal route, immediately before, and 1 hour after induction of the ulcer. The animals were killed 4 hours after injection of reserpine or of serotonin, and the gastric lesions were then assessed.

2. In the case of the groups with ulcers induced by phenylbutazone, the product was administered at doses of from 1 to 4 mg/kg, in physiological saline (10 ml/kg); in parallel, the control group received physiological saline (10 ml/kg).

Administration was by the intraperitoneal route, starting the treatment 6 hours after injection of the phenylbutazone and then once per day for 5 consecutive days.

The animals were killed 6 hours after the last dose of product, then the stomachs were removed, opened along the greater curvature and examined with a magnifying glass.

Results

In the ulcers induced by reserpine or by serotonin, the product exerts a pronounced protective action, which is manifested in a dose-dependent manner in the interval tested, with a maximum percentage of inhibition of about 68% in the case of reserpine ulcers, and of about 51% in the case of serotonin ulcers, relative to the controls.

This demonstrates a positive action of the product on the microcirculation. It is probable that the capillary-protective activity is correlated, at least in part, with an anti-serotonin action.

In the ulcers induced by phenylbutazone, the product is found to have a pronounced healing action, of a dose-dependent type, in the interval tested, with a maximum percentage inhibition of about 59%, relative to the controls.

The fraction SF has been shown to possess, in vivo as well as otherwise, pronounced re-epithelializing and curative properties on wounds and/or ulcers of whatever nature, including gastric ulcers. It shows notable activation of cellular growth in fibroblasts, and of the activity of ODC.

Activation of the mechanism of hydrolysis of inositol phospholipids, typical of growth factors, was also demonstrated.

The doses and dosages should be prescribed by the attending physician and can vary according to the type of pathology to be treated, its extent, the conditions of the patient (age, sex, weight) and the type of pharmaceutical composition. As a guide, the doses (based on the pure and dried fraction SF) can vary from 0.5 $\mu$g/kg to 5 $\mu$g/kg.

The fraction according to the present invention can be administered in the form of a pharmaceutical composition containing a therapeutically effective quantity of the said fraction mixed with conventional vehicles and excipients known to the person skilled in the art.

The compositions according to the present invention can be prepared by known methods and techniques, as described for example in "Remington's Pharmaceutical Sciences Handbook", latest edition, Mack Pub. Inc., N.Y., USA.

Other active principles that can be used for the indications as above can also be present.

Examples of pharmaceutical compositions are ampoules, creams, dusting powders, ointments, tablets, capsules, oral liquid forms, suppositories, injectable liquid forms.

The doses (based on the pure and dried fraction SF) can vary according to the composition; as a guide, they can vary from 2 mg to 20 mg per 100 g of total composition or from 35 $\mu$g to 350 $\mu$g per single dose.

According to the present invention, the fraction described, obtained from starch, can also be used for the cosmetic treatment of wounds and scars. Hence, the present invention also comprises the use of the said fraction for the cosmetic treatment of the skin, as well as cosmetic compositions containing an effective amount of the said fraction. The cosmetic compositions can be prepared by conventional methods known to the person skilled in the art.

The invention is further illustrated by the following example.

**EXAMPLE**

5 kg of starch are placed in 50 litres of an aqueous solution containing 0.6 mM HCl and 1.8 mM $H_2SO_4$.

Hydrolysis is effected in an autoclave for 1 hour at 1 atmosphere (approx. 121°C), the whole being placed in hermetically sealed glass flasks.

The supernatant is mixed with a sufficient quantity of filter aid ("Clarcel CB" diatomaceous earth) and filtered in a filter-press on board filters, the filters being washed with distilled water until the initial volume is obtained; the pH is corrected to the range 3.5-4.5 with 4N NaOH.

This is diluted to obtain a predetermined total dry yield (1.0%), and is then sterilized.

The product thus obtained is called the starting extract.

In an ultrafiltration unit of appropriate dimensions, the molecules with molecular weight greater than 30 000 daltons are eliminated by using an ultrafiltration membrane with cut-off at 30 000 daltons.

This operation is halted when the volume of retained material is about one tenth of the starting volume; the first eluate (E1) is collected.

The retained material is diluted again to the initial volume with distilled water and the ultrafiltration process is repeated; the second eluate (E2) is combined with the first eluate (E=E1+E2) and designated <30, whereas the retained material is eliminated.

The eluate <30, obtained from the preceding stage, is treated in the same ultrafiltration plant, using an ultrafiltration membrane with cut-off at 3000 daltons.

The operation is halted when the volume of the retained material is one tenth of the starting volume, and the eluate is discarded. The retained material is diluted with distilled water until it has the starting volume and the process is repeated; this is done at least 4-5 times.

Finally the residue is concentrated, still by means of the same membrane, down to the minimum volume allowed by the apparatus. Filtration is finally effected through a 0.22-micron sterilizing membrane.

The resulting material is lyophilized, if necessary using a solid support such as lactose (or mannitol) in a ratio by weight (based on dry matter) of 5-10 between support and SF.

**Claims**

1. Process for the preparation of a starch fraction that has a molecular weight between 3000 and 30 000, which comprises:

   a) hydrolysis of the starch in an aqueous solution at a pH of between 2 and 7 at a temperature between 100 and 150°C;
   b) elimination of the solid residue until a clear first solution is obtained;
   c) elimination of the fraction with molecular weight greater than 30 000 daltons from the said first solution, to give a second solution;
   d) isolation of the fraction with molecular weight between 3000 and 30 000 from the said second solution.

2. Process according to Claim 1, characterized in that stage a) is carried out at a pH of between 2 and 4.

3. Process according to Claim 1 or 2, characterized in that the solution obtained from stage b) has whole starch added to it and is subjected to stage a).

4. Process according to Claims 1-3, characterized in that stages c) and d) are effected by means of ultrafiltration.

5. Starch fraction obtainable by means of the process according to Claims 1-4.

6. Fraction according to Claim 5, characterized in that it gives a chromatogram as illustrated in Fig. 1.

7. Use of the fraction according to Claim 5 as active principle for the preparation of a medicament that can be used for the treatment of wounds and ulcers.

8. Pharmaceutical compositions containing a therapeutically effective amount of the fraction of Claim 5 mixed with conventional vehicles and excipients.

9. Compositions according to Claim 8, in the form of ampoules, creams, tablets.

10. Use of the fraction of Claim 5 for the cosmetic treatment of the skin.

**11.** Cosmetic compositions containing an effective amount of the fraction of Claim 5 mixed with conventional vehicles and excipients.

FIGURE 1

FIGURE 2

| | European Patent Office | EUROPEAN SEARCH REPORT | | Application Number |
| | | | | EP 95 10 7578 |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | US-A-3 812 252 (SILVETTI)<br>* column 1, line 12 - line 18 *<br>* column 3, line 1 - line 7 *<br>* column 3, line 42 - line 60 *<br>* column 4, line 23 - line 30 *<br>--- | 1,5,7-9 | C08B30/18<br>A61K31/715<br>A61K7/48 |
| A | GB-A-1 418 910 (GORDON GERARD BIRCH)<br>* claims 1,4 *<br>--- | 1 | |
| A | US-A-3 668 007 (C. T. EGGER ET AL.)<br>* column 2, line 15 - line 48 *<br>--- | 1 | |
| A | DD-A-240 208 (AKADEMIE DER WISSENSCHAFTEN DES DDR)<br>* claims *<br>--- | 1,4 | |
| A | FR-A-2 309 638 (MULLER H.)<br>* page 2, line 7 - line 18; claims 1-5 *<br>----- | 1,4 | |
| | | | TECHNICAL FIELDS SEARCHED (Int.Cl.6)<br><br>C08B<br>A61K<br>C13K |

The present search report has been drawn up for all claims

| Place of search | Date of completion of the search | Examiner |
|---|---|---|
| THE HAGUE | 10 October 1995 | Mazet, J-F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)